# EUROPÄISCHE PATENTSCHRIFT

(11) **EP 1 076 654 B1**
(45) Veröffentlichungstag und Bekanntmachung des Hinweises auf die Patenterteilung: **25.06.2003**
(21) Anmeldenummer: 99923434.7
(22) Anmeldetag: 22.04.1999
(51) Int. Cl.: C07D 317/36, C07D 317/38

(54) **VERFAHREN ZUR KONTINUIERLICHEN HERSTELLUNG VON 1,3-DIOXOLAN-2-ONEN**
METHOD FOR THE CONTINUOUS PRODUCTION OF 1,3-DIOXOLAN-2-ONES
PROCEDE DE PREPARATION CONTINUE DE 1,3-DIOXOLANE-2-ONES

(30) Priorität: 30.04.1998 DE 19819586
(43) Veröffentlichungstag der Anmeldung: 21.02.2001
(73) Patentinhaber: BASF AKTIENGESELLSCHAFT, 67056 Ludwigshafen (DE)
(72) Erfinder: BIRNBACH, Stefan, D-67246 Dirmstein (DE); DOCKNER, Toni, D-67149 Meckenheim (DE); MOHR, Jürgen, D-67269 Grünstadt (DE); BENFER, Regina, D-67122 Altrip (DE); BIEG, Walter, D-67269 Grünstadt (DE); PETERS, Jarren, D-68163 Mannheim (DE); RUGE, Bernd, D-67459 Böhl-Iggelheim (DE); WEINLE, Werner, D-67159 Friedelsheim (DE); ZEHNER, Peter, D-67071 Ludwigshafen (DE)
(86) Internationale Anmeldenummer: EP9902701
(87) Internationale Veröffentlichungsnummer: WO99057108

(56) Entgegenhaltungen:
- EP-A- 0 069 494
- EP-A- 0 543 249
- DE-B- 1 169 459
- US-A- 2 773 070
- W. J. PEPPEL: INDUSTRIAL AND ENGINEERING CHEMISTRY, Bd. 50, Nr. 5, 1958, Seiten 767-70, XP002123009 in der Anmeldung erwähnt

## Beschreibung

Die vorliegende Erfindung betrifft ein verbessertes Verfahren zur kontinuierlichen Herstellung von 1,3-Dioxolan-2-onen wie Ethylencarbonat oder Propylencarbonat aus den entsprechenden Oxiranen und Kohlendioxid in flüssiger Phase in Gegenwart eines Katalysators.

In der Literaturstelle Ind. Eng. Chem. 50 (1958), S. 767 bis 770, wird ein Verfahren zur Herstellung von Ethylencarbonat beschrieben, bei dem die Umsetzung des Ethylenoxids mit dem Kohlendioxid in einem Rohrreaktor erfolgt, an dessen Kopf Ethylenoxid und Katalysatorlösung bei einer Temperatur von 155°C eingespeist und an dessen Fuß bei einer Temperatur von 195°C das Kohlendioxid zugefügt und das erzeugte Ethylencarbonat ausgeschleust werden. In einem nachgeschalteten separaten Kessel wird eine Nachreaktion durchgeführt. Im Reaktor herrscht ein Druck von 1500 psig (103 bar). Er kann kontinuierlich betrieben werden. Das Kohlendioxid wird im Überschuß angeboten. Bei dem im Reaktor vorliegenden Temperaturgradienten von 40°C kann nicht von rückvermischenden Bedingungen gesprochen werden. Im Nachreaktor wird ein Umsatz von knapp unter 99 % erreicht, wobei allerdings eine Reihe von Nebenprodukten gebildet werden; die Ausbeute an Ethylencarbonat liegt somit bei ca. 93 %.

Aufgabe der vorliegenden Erfindung war es, ein verbessertes Verfahren zu entwickeln, das einen besonders hohen Umsatz des Oxirans, insbesondere von mindestens 99,9 %, bei gleichzeitiger hoher Selektivität und hoher Raum-Zeit-Ausbeute erlaubt. Auch soll das Produkt eine so hohe Reinheit aufweisen, daß Nebenprodukte nur im ppm-Bereich auftreten. Der gewünschte hohe Umsatz wird vor allem dadurch erreicht werden, daß im Sinne der vorliegenden Erfindung ein zweiteiliger Reaktor mit Gegenstromfahrweise zwischen Oxiran und Kohlendioxid verwendet wird.

Demgemäß wurde ein Verfahren zur kontinuierlichen Herstellung von 1,3-Dioxolan-2-onen der allgemeinen Formel I 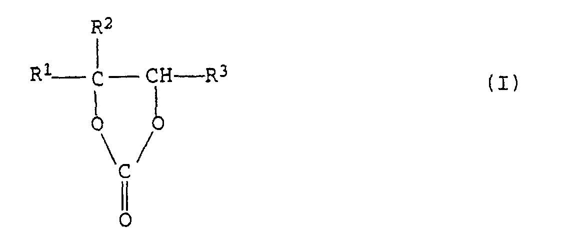 in der R¹ für Wasserstoff oder einen organischen Rest mit bis zu 40 C-Atomen steht und R² und R³ jeweils Wasserstoff oder eine C₁bis C₄-Alkylgruppe bedeuten, wobei R² und R³ auch miteinander zu einem fünf- oder sechsgliedrigen Ring verbunden sein können,
durch Umsetzung eines Oxirans der allgemeinen Formel II 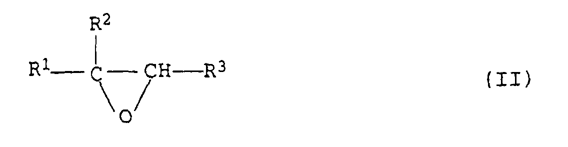 mit Kohlendioxid in flüssiger Phase in Gegenwart eines Katalysators gefunden, welches dadurch gekennzeichnet ist, daß man die Umsetzung in einem zweiteiligen Reaktor durchführt, bei dem im ersten Teil die Umsetzung unter Rückvermischung bis zu einem Umsatz des Oxirans II von mindestens 80 %, insbesondere mindestens 90 %, geführt und im zweiten Teil die Umsetzung unter nicht rückvermischenden Bedingungen vervollständigt wird, wobei das Kohlendioxid im gesamten Reaktor im Gegenstrom zum Oxiran II geführt wird.

Die beiden Teile des Reaktors können jeweils aus einer Stufe oder aus mehreren hintereinander oder parallel geschalteten Stufen bestehen. Der gesamte Reaktor kann bautechnisch aus einem oder aus mehreren Apparaten bestehen. Zweckmäßigerweise führt man dabei das frische Oxiran II sowie den frischen Katalysator dem ersten Teil des Reaktors und das frische Kohlendioxid dem zweiten Teil des Reaktors zu.

Es ist für den Erfolg des Verfahrens von Bedeutung, für eine ausreichende Rückvermischung innerhalb des ersten Teils des Reaktors zu sorgen. Durch die spezielle Bauart des ersten Teils des Reaktors wird die Rückvermischung gefördert oder erst ermöglicht, beispielsweise durch die Wahl eines Schlaufenreaktors. Weiterhin können auch externe Rückvermischungen, etwa durch das Zurückpumpen eines Teils des Produktstroms, welcher den ersten oder den zweiten Teil des Reaktors verläßt, in den ersten Teil des Reaktors, vorteilhaft eingesetzt werden.

Zweckmäßigerweise führt man die Umsetzung im ersten Teil des Reaktors isotherm unter Abfuhr der Reaktionswärme durch. Da die Umsetzung der Oxirane II zu den 1,3-Dioxolan-2-onen I meist stark exotherm ist, ist eine effektive Wärmeabfuhr notwendig. In der Regel führt man die Reaktionswärme durch einen internen oder externen Wärmeaustauscher mit einem Volumenstrom vom 30fachen bis 500fachen des Durchsatzes durch den Umsetzungsreaktor ab. In der Regel liegen die Temperaturschwankungen im ersten Teil des Reaktors bei maximal ± 5°C, insbesondere bei maximal ± 3°C.

Der Druck, mit dem der Reaktor betrieben wird, liegt in beiden Teilen des Reaktors normalerweise bei 2 bis 50 bar, insbesondere bei 5 bis 40 bar, vor allem bei 10 bis 30 bar. Durch diese relativ niedrigen Drücke gestaltet sich das erfindungsgemäße Verfahren vom apparativen Aufwand her meist unkompliziert.

Ein weiterer wesentlicher Faktor für den wirtschaftlichen Erfolg des erfindungsgemäßen Verfahrens ist die Einhaltung eines bestimmten Temperaturbereichs in beiden Teilen des Reaktors. Die Temperatur sollte an keiner Stelle des Reaktors über 150°C liegen. Bevorzugte Temperaturbereiche sind 70 bis 150°C, insbesondere 90 bis 145°C, vor allem 100 bis 140°C. Im zweiten Teil des Reaktors kann die Temperatur bis zu 40°C, insbesondere bis zu 25°C, vor allem bis zu 10°C, über der Temperatur, die im ersten Teil des Reaktors herrscht, liegen,

Durch einen hohen Umsatz von mindestens 80 %, vorzugsweise mindesten 90 %, insbesondere mindestens 95 %, vor allem von mindestens 98 %, im ersten Teil des Reaktors und die gute Rückvermischung in diesem Reaktorteil kann in aller Regel gewährleistet werden, daß an keiner Stelle im Reaktor Oxirankonzentrationen über 20 Gew.-%, vorzugsweise über 10 Gew.-%, insbesondere über 5 Gew.-%, vor allem über 2 Gew.-%, vorliegen. Dieses hat sowohl sicherheitstechnische Relevanz (Ethylenoxid!) als auch wesentlichen Einfluß auf die Selektivität. So wurde gefunden, daß bei erhöhter Oxirankonzentration vermehrt Nebenprodukte gebildet werden.

Der erste Teil des Reaktors arbeitet aufgrund einer hohen adiabatischen Temperaturerhöhung vorzugsweise isotherm. Die Reaktionswärme kann je nach Bauart des Reaktors durch einen internen oder externen Wärmeaustauscher abgeführt werden. Die gute Rückvermischung im Reaktor verhindert das Auftreten von unerwünschten Temperaturspitzen. Außerdem ist es zur Unterstützung der Reaktion vorteilhaft, das erfindungsgemäß im Gegenstrom zum Oxiran II zugeführte gasförmige Kohlendioxid in jedem Reaktorteil fein zu dispergieren, um hohe Stoffaustauschflächen zu erhalten. Normalerweise liegt das Kohlendioxid gesättigt in der Flüssigphase vor. Dadurch wird sichergestellt, daß im gesamten Reaktor genügend Kohlendioxid zur Verfügung steht. Besonders vorteilhaft ist im Sinne der vorliegenden Erfindung bei der Gegenstromfahrweise, daß nicht umgesetztes Oxiran aus dem zweiten Reaktorteil in den ersten Teil gestrippt wird, in der der Hauptumsatz erfolgt. Diese Technik reduziert die Austrittskonzentration an Oxiran im erzeugten Alkylencarbonat und begünstigt damit einen hohen Umsatz.

Der obere Teil des Reaktors stellt bautechnisch in der Regel den ersten, rückvermischten Reaktorteil dar. Bevorzugt wird die Vermischung in diesem Reaktorteil durch einen Flüssigkeitsstrahl erreicht. Zur Unterstützung der Vermischung und Begasung können Einbauten im Reaktor, wie z.B. ein Leitrohr oder ein Impulsaustauschrohr, angebracht sein. Die Dispergierung der Gasphase in diesem Reaktorteil kann über den Flüssigkeitsstrahl oder eine fein verteilte Gaszufuhr am Boden erfolgen. Der Katalysator und das frische Oxiran können an beliebiger Stelle diesem Reaktorteil zugeführt werden. Bevorzugt wird ein Eintrag des Oxirans in den Flüssigkeitsstrahl, so daß es in der Flüssigkeit gleichmäßig verteilt vorliegt.

Der Katalysator gelangt mit dem Flüssigkeitsaustrag der ersten Stufe in den zweiten Reaktorteil. Der Flüssigkeitsaustrag aus dem ersten Teil kann an beliebiger Stelle, bevorzugt jedoch am Boden, erfolgen. Am Kopf des Reaktors können Inerte (Edelgase, Stickstoff) und nicht umgesetztes Kohlendioxid dem Reaktionsgemisch entnommen werden. Ein Teilstrom des Abgases kann zusammen mit dem Frischgas wieder dem zweiten Reaktorteil zugeführt werden.

Der zweite Teil des Reaktors kann als mehrstufiger im Gegenstrom betriebener Gas- und Flüssig-Kontaktapparat ausgeführt sein (gekühlt oder adiabatisch betrieben). Bevorzugt wird die Ausführung als Blasensäulenkaskade, aber auch andere Bauformen, z.B. eine Rührkessel-Kaskade, sind möglich. Die Einteilung dieses Reaktorteils in mehrere Stufen kann durch Einbauten wie Lochbleche, Glockenböden oder spezielle Mischelemente oder durch Verwendung mehrerer Apparate erfolgen. Dabei sollte das Gas möglichst gut in jeder Stufe dispergiert werden, wozu sich entsprechende statische oder dynamische Gasverteiler wie Lochböden, Düsen oder Rührelemente eignen. Ebenso ist ein Festbett mit Rohrreaktor-Charakteristik bestehend aus Füllkörpern bzw. gepackten Einbauten, eventuell bereits mit aufgezogenem Katalysator, möglich. In diesem Fall ist das Kohlendioxid stationäre Phase und die Reaktionslösung bildet einen Film auf den Einbauten, wodurch ebenfalls eine große Stoffaustauschfläche geschaffen wird. Denkbar ist auch, das Frischgas Kohlendioxid verteilt über die einzelnen Stufen des zweiten Reaktorteils zuzugeben.
Bevorzugt wird jedoch der vollständige Eintrag in der letzten Stufe. Um eine Strömungsführung wie oben erläutert zu erhalten, kann es erforderlich sein, die beiden Reaktorteile und die einzelnen Stufen mit verschiedenen Druckniveaus (im Rahmen des für den gesamten Umsetzungsreaktor vorgegebenen Bereiches) zu betreiben. Vorzugsweise ist hierbei das höchste Druckniveau in der letzten Stufe.

Das Umsetzungsgemisch mit dem erzeugten Verfahrensprodukt I wird aus der letzten Reaktorstufe kontinuierlich ausgetragen.

Als Katalysatoren für das erfindungsgemäße Verfahren kommen praktisch alle aus der Literatur, z.B. aus US-A 2 773 070, US-A 2 773 881, Chem. Lett. (1979) S. 1261, Chem. Lett (1977) S. 517, DE-A 35 29 263, DE-B 11 69 459, EP-A 069 494 oder EP-B 543 249, für derartige Umsetzungen bekannten Katalysatoren in Betracht. Mit besonderem Vorteil setzt man jedoch als Katalysatoren Oniumsalze oder Metallsalze oder Mischungen hieraus ein.

Als Oniumsalze eignen sich prinzipiell alle Verbindungen dieses Typs, darunter besonders Ammonium-, Phosphonium- und Sulfoniumsalze der allgemeinen Formeln IIIa bis IIIc 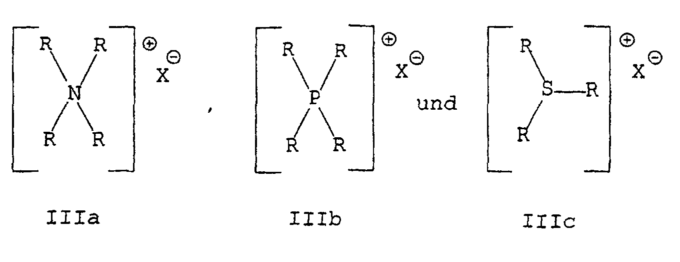 in denen die Substituenten R gleiche oder verschiedene Kohlenwasserstoffreste mit jeweils 1 bis 20 C-Atomen bedeuten, wobei die Summe der C-Atome in den Resten R jeweils nicht größer als 24 ist, und in denen X vor allem Halogen, vorzugsweise Brom oder Iod, bedeutet.

Wegen der generellen Eignung der Oniumsalze richtet sich deren Wahl hauptsächlich nach deren Verfügbarkeit und nach deren Preis. Praktisch wird man daher vor allem mit den Ammoniumsalzen IIIa arbeiten, wobei das handelsübliche und leicht herzustellende Tetraethylammoniumbromid an erster Stelle zu nennen ist. Daneben sind diejenigen Verbindungen IIIa hervorzuheben, in denen drei der Reste R niedere Alkylgruppen wie die Methyl-oder Ethylgruppe und der vierte den Benzylrest oder einen unverzweigten C₆- bis C₁₈-Alkylrest bedeuten.

Unter den Phosphoniumsalzen IIIb sind diejenigen am besten zugänglich, die sich vom Triphenylphosphin ableiten und deren vierter Substituent durch Quaternierung mit einem C₁- bis C₆-Alkylbromid in das Molekül eingeführt wurde.

Als geeignetes Sulfoniumsalz IIIc sei beispielsweise das leicht herstellbare Trimethylsulfoniumiodid genannt. Im allgemeinen sind die Ammonium- und Phosphoniumsalze indes besser geeignet als die Sulfoniumsalze.

Allgemein können die Kohlenwasserstoffreste R in den Verbindungen IIIa bis IIIc verzweigte oder vorzugsweise unverzweigte C₁- bis C₂₀-Alkylgruppen, Aralkylgruppen wie die Benzylgruppen, die Cyclohexylgruppe und aromatische Gruppen wie die Phenyl-oder die p-Tolylgruppe bedeuten. Ferner können Alkylreste R auch miteinander verbunden sein, etwa unter Ausbildung eines Piperidinringes. Als Anionen kommen außer Halogen beispielsweise Sulfat und Nitrat in Betracht.

Häufig, und zwar besonders im Falle der Oniumbromide, ist es nicht erforderlich, von den Salzen IIIa bis IIIc selber auszugehen, sondern es genügt, deren Vorprodukte Base und Quaternierungsreagens einzusetzen, aus denen sich die wirksamen Quatenierungsprodukte IIIa bis IIIc von selber bilden.

Als Metallsalze kommen Salze von Alkalimetallen, Erdalkalimetallen und Übergangsmetallen, insbesondere von zweiwertigen Übergangsmetallen, in Betracht, beispielsweise Natrium-, Kalium-, Magnesium-, Calcium-, Aluminium-, Mangan(II)-, Eisen(II)-, Nickel(II)-, Kupfer(II)-, Zink-, Cadmium- oder Blei(II)-Salze. Als Anionen für diese Salze eignen sich Sulfat, Nitrat, Phosphat, Carbonat, Acetat, Formiat sowie vor allem Halogenide wie Chlorid, Bromid und Iodid. Besonders gute Resultate erzielt man mit Zinksalzen wie Zinksulfat, Zinknitrat, Zinkphosphat, Zinkcarbonat, Zinkacetat, Zinkformiat, Zinkchlorid, Zinkbromid oder Zinkiodid. Man kann selbstverständlich auch Mischungen derartiger Metallsalze einsetzen, das gleiche gilt auch für die oben genannten Oniumsalze. Auch Mischungen von Oniumsalzen mit Metallsalzen sind möglich und zeigen in einigen Fällen sogar überraschende, Vorteile.

Die Menge der als Katalysatoren eingesetzten Oniumsalze und/oder der Metallsalze ist im Prinzip beliebig, da sie offensichtlich lediglich einen Einfluß auf die Reaktionsgeschwindigkeit hat, deren Größenordnung sich ihrerseits nach der Art des eingesetzten Oxirans II richtet. Für die technisch befriedigenden Reaktionszeiten von etwa 0,5 bis 5 h benötigt man in der Regel etwa 0,01 bis 3 Gew.-%, bezogen auf eingesetztes Oxiran II.

In einer bevorzugten Ausführungsform setzt man als Katalysatoren Alkalimetallbromide, Alkalimetalliodide, Tetraalkylammoniumbromide, Tetraalkylammioniumiodide, Halogenide zweiwertiger Metalle oder Mischungen hieraus ein.

In einer ganz besonders bevorzugten Ausführungsform setzt man als Katalysatoren eine Mischung aus Oniumsalzen, insbesondere Ammonium-, Phosphonium- und/oder Sulfoniumsalzen der allgemeinen Formel IIIa bis IIIc, und Zinksalzen, insbesondere den oben explizit genannten, ein. Die wirksamen Mengen der Zinksalze liegen hierbei je nach der Reaktivität des eingesetzten Oxirans, der Aktivität des Oniumsalzes und den sonstigen Reaktionsbedingungen zwischen 0,1 bis 1,0 mol, vorzugsweise zwischen 0,3 und 0,7 mol, pro Mol des Oniumsalzes. Im Falle der Verwendung von Zinkbromid betragen diese Mengen im allgemeinen 0,2 bis 0,8 mol, vorzugsweise 0,3 bis 0,5 mol, pro Mol des Oniumsalzes.

Das erfindungsgemäße Verfahren kann ohne Lösungsmittel durchgeführt werden, jedoch kann ein inertes Lösungsmittel wie Dioxan, Toluol oder Aceton, normalerweise in Mengen von etwa 10 bis 100 Gew.%, bezogen auf das eingesetzte Oxiran II, mitverwendet werden. Ist das Verfahrensprodukt I unter den Reaktionsbedingungen flüssig, verwendet man zweckmäßigerweise dieses als Lösungsmittel. Es hat sich in solchen Fällen als günstig erwiesen, den Katalysator in dem Verfahrensprodukt zu lösen und diese Lösung zuzudosieren, wobei praktisch keine weiteren Lösungsmittel im Umsetzungsreaktor zugegen sind. Hierbei beträgt die Konzentration des Katalysators im Verfahrensprodukt I üblicherweise 0,5 bis 20 Gew.-%, insbesondere 1 bis 15 Gew.-%; das molare Verhältnis von in der gleichen Zeiteinheit zugegebener Eduktmenge II zu mit dem Katalysator zugegebenem Verfahrensprodukt I liegt in der Regel bei 100:1 bis 1:1, insbesondere 50:1 bis 2:1.

Beim erfindungsgemäßen Verfahren setzt man die Eduktströme an Oxiran II und Kohlendioxid vorzugsweise im molaren Verhältnis von 1:1 bis 1:1,05, insbesondere 1:1 bis 1:1,02, ein. Ein möglicher leichter Überschuß an Kohlendioxid ist durch die Verluste an Kohlendioxid beim Entspannen des Druckes bedingt.

Normalerweise erzielt man mit dem erfindungsgemäßen Verfahren praktisch quantitative Umsätze an II, in der Regel mindestens 99 %, insbesondere mindestens 99,5 %, vor allem mindestens 99,9 %, wonach man das Reaktionsgemisch im Prinzip wie üblich aufarbeitet. Hierbei anfallende katalysatorhaltige Rückstände können meist mehrmals für weitere Reaktionsansätze verwendet werden, bis ein eingesetztes Metallsalz infolge der nie ganz auszuschließenden Spuren von Wasser durch Hydrolyse desaktiviert worden sind.

Um ein hochreines Produkt zu erhalten, ist bei geeigneter Kombination aus Reaktionsbedingungen und Katalysatorart und -konzentration in der Regel nur eine Abtrennung des Katalysators und des nicht umgesetzten Oxirans erforderlich. Die Abbrennung und Rückführung des Katalysators ist auch aus wirtschaftlichen Gesichtspunkten empfehlenswert. Die Aufarbeitung des Produktes kann auf mehrere Weisen erfolgen. Im allgemeinen ist es erforderlich, zunächst durch Entspannung das gelöste Kohlendioxid zu entfernen. Sowohl zur Katalysatorabtrennung als auch zur Oxiranentfernung können die üblichen verfahrenstechnischen Methoden wie Destillation, Extraktion oder Strippen eingesetzt werden.

Nach den bisherigen Beobachtungen ist das gute Gelingen des erfindungsgemäßen Verfahrens von der Art des eingesetzten Oxirans (Epoxids) II nicht abhängig, sofern der Rest R¹, wie sich allerdings von selbst versteht, keine sauren Substituenten enthält, welche unter Öffnung des Oxiranringes zu Nebenreaktionen Anlaß geben.

Als Reste R¹ seinen neben Wasserstoff genannt:
- gesättigte und ungesättigte verzweigte und unverzweigte aliphatische Reste mit bis zu 40 C-Atomen, insbesondere bis zu 18 C-Atomen,
- iso- oder heterocyclische cycloaliphatische Gruppen mit vorzugsweise 5 bis 7 Ringgliedern,
- iso- oder heterocyclische aromatische Gruppen und
- gemischte Reste mit Gruppierungen der vorgenannten Art, beispielsweise araliphatische Reste wie die Benzylgruppe.

Die Gruppe können Substituenten wie Halogen, Nitrogruppen, freie oder substituierte Aminogruppen, Hydroxylgruppen, Formylgruppen oder Cyangruppen tragen oder Ether-, Keton- oder Estergruppierungen enthalten.

Bei den Resten R² und R³ handelt es sich in der Regel um Wasserstoff oder die Methylgruppe sowie um solche Reste, die miteinander zu einem fünf- oder sechsgliedrigen Ring verbunden sind, wofür Cyclohexenoxid als Verbindung II ein Beispiel ist. Enthält II zwei Oxiranringe mit jeweils einer (CH₂-)-Gruppierung, so enthält man die entsprechenden Bis-Dioxolane I. Oxiranringe, die an beiden C-Atomen substituiert sind, werden in der Regel langsamer angegriffen als solche, die nur an einem der C-Atome substituiert sind.

In einer bevorzugten Ausführungsform stellt man mit dem erfindungsgemäßen Verfahren 1,3-Dioxolan-2-one der allgemeinen Formel I her, in der der Rest R¹ für Wasserstoff oder eine C₁₋ bis C₁₈-Alkylgruppen, insbesondere eine C₁₋ bis C₄-Alkylgruppe, steht. Das erfindungsgemäße Verfahren eignet sich in ganz besonderem Maße zur Herstellung von Ethylencarbonat oder Propylencarbonat, daneben aber auch zur Herstellung der entsprechenden Carbonate aus Isobutylenoxid, Styroloxid oder Epichlorhydrin.

Die Verfahrensprodukte I sind wertvolle Zwischenprodukte für organische Synthesen. Darüber hinaus finden Ethylen- und Propylencarbonat in größerem Umfang Verwendung als Lösungsmittel, vor allem in der Synthesefaserindustrie.

### Beispiel 1 - Herstellung von Propylencarbonat

Der verwendete Reaktor wies einen ersten (oberen) Reaktorteil (Schlaufenreaktor) mit einem externem Kreislaufstrom mit externem Wärmeaustauscher, über den die Reaktionstemperatur auf 120°C ± 2°C gehalten wurde, und einen zweiten (unteren) Reaktorteil, bestehend aus einer fünfstufigen Gegenstromblasensäulenkaskade, die bei Temperaturen von 122°C (in der obersten Stufe) bis 130°C (in der untersten Stufe) arbeitete, auf. Der Reaktor wurde in allen Teilen mit einem Druck von 16 bar betrieben.

In den oberen Reaktorteil wurden 2,00 kg/h Propylenoxid sowie 0,456 kg/h einer Lösung von 12 Gew.-% eines Zinkbromid/Tetraethylammoniumbromid-Gemisches im Gew.-Verhältnis 1 zu 2 als Katalysator in Propylencarbonat gelöst kontinuierlich eingespeist. Am Boden der untersten Stufe der Blasensäulenkaskade wurden 1,52 kg/h Kohlendioxid kontinuierlich zugeführt und gleichzeitig 4,00 kg/h Produkt kontinuierlich entnommen. Das entnommenen Propylencarbonat gelöst wies einen Gehalt an Proylenoxid von nur ca. 200 ppm auf, Nebenprodukte wie Aceton und Propionaldehyd waren in einer Konzentration von deutlich weniger als 200 ppm vorhanden. Der Umsatz, bezogen auf eingesetztes Propylenoxid, betrug 99,9 %.

### Beispiel 2 - Herstellung von Ethylencarbonat

Es wurden der gleiche Reaktor und die gleichen Reaktionsparameter wie in Beispiel 1 verwendet.

In die obere Reaktorstufe wurden 1,52 kg/h Ethylenoxid sowie 0,27 kg/h einer Lösung von 12 Gew.-% eines Zinkbromid/Tetraethylammoniumbromid-Gemisches im Gew.-Verhältnis 1 zu 2 als Katalysator in Ethylencarbonat gelöst kontinuierlich eingespeist. Am Boden der untersten Stufe der Blasensäulenkaskade wurden 1,52 kg/h Kohlendioxid kontinuierlich zugeführt und gleichzeitig 3,30 kg/h Produkt kontinuierlich entnommen. Das entnommene Ethylencarbonat wies einen Gehalt an Ethylenoxid von weniger als 100 ppm auf, Nebenprodukte wie Acetaldehyd waren in einer Konzentration von nur ca. 10 ppm vorhanden. Der Umsatz, bezogen auf eingesetztes Ethylenoxid, betrug 99,95%.

## Patentansprüche

1. Verfahren zur kontinuierlichen Herstellung von 1,3-Dioxolan-2-onen der allgemeinen Formel I 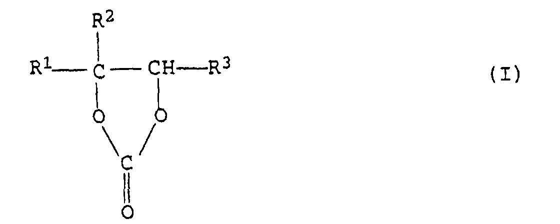 in der R¹ für Wasserstoff oder einen organischen Rest mit bis zu 40 C-Atomen steht und R² und R³ jeweils Wasserstoff oder eine C₁- bis C₄-Alkylgruppe bedeuten, wobei R² und R³ auch miteinander zu einem fünf- oder sechsgliedrigen Ring verbunden sein können,
durch Umsetzung eines Oxirans der allgemeinen Formel II 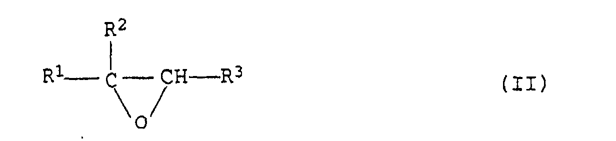 mit Kohlendioxid in flüssiger Phase in Gegenwart eines Katalysators, **dadurch gekennzeichnet, daß** man die Umsetzung in einem zweiteiligen Reaktor durchführt, bei dem im ersten Teil die Umsetzung unter Rückvermischung bis zu einem Umsatz des Oxirans II von mindestens 80 %, insbesondere mindestens 90 %, geführt und im zweiten Teil die Umsetzung unter nicht rückvermischenden Bedingungen vervollständigt wird, wobei das Kohlendioxid im gesamten Reaktor im Gegenstrom zum Oxiran II geführt wird.

2. Verfahren nach Anspruch 1, **dadurch gekennzeichnet, daß** man das frische Oxiran II sowie den frischen Katalysator dem ersten Teil des Reaktors und das frische Kohlendioxid dem zweiten Teil des Reaktors zuführt.

3. Verfahren nach Anspruch 1 oder 2, **dadurch gekennzeichnet, daß** man die Umsetzung im ersten Teil des Reaktors isotherm unter Abfuhr der Reaktionswärme durchführt.

4. Verfahren nach Anspruch 3, **dadurch gekennzeichnet, daß** man die Reaktionswärme durch einen internen oder externen Wärmeaustauscher mit einem Volumenstrom vom 30fachen bis 500fachen des Durchsatzes durch den Reaktor abführt.

5. Verfahren nach den Ansprüchen 1 bis 4, **dadurch gekennzeichnet, daß** man im zweiten Reaktorteil die Umsetzung bis zu einem Umsatz des Oxirans II von mindestens 99 %, insbesondere mindestens 99,5 %, vervollständigt.

6. Verfahren nach den Ansprüchen 1 bis 5, **dadurch gekennzeichnet, daß** man den Reaktor in beiden Teilen bei einem Druck von 2 bis 50 bar, insbesondere von 5 bis 40 bar, betreibt.

7. Verfahren nach den Ansprüchen 1 bis 6, **dadurch gekennzeichnet, daß** man den Reaktor in beiden Teilen bei Temperaturen von 70 bis 150°C, insbesondere von 90 bis 145°C, betreibt.

8. Verfahren nach den Ansprüchen 1 bis 7, **dadurch gekennzeichnet, daß** man das Oxiran II und das Kohlendioxid im molaren Verhältnis von 1:1 bis 1:1,05 einsetzt.

9. Verfahren nach den Ansprüchen 1 bis 8, **dadurch gekennzeichnet, daß** man als Katalysatoren Oniumsalze oder Metallsalze oder Mischungen hieraus einsetzt.

10. Verfahren nach Anspruch 9, **dadurch gekennzeichnet, daß** man als Katalysatoren Alkalimetalliodide, Alkalimetallbromide, Tetraalkylammoniumiodide, Tetraalkylammoniumbromide, Halogenide zweiwertiger Metalle oder Mischungen hieraus einsetzt.

11. Verfahren nach den Ansprüchen 1 bis 10 zur Herstellung von 1,3-Dioxolan-2-onen I, in der der Rest R¹ für Wasserstoff oder eine C₁- bis C₁₈-Alkylgruppe steht.

12. Verfahren nach Anspruch 11 zur Herstellung von Ethylencarbonat oder Propylencarbonat.

## Claims

1. A process for continuous production of 1,3-dioxolan-2-ones of the general formula I 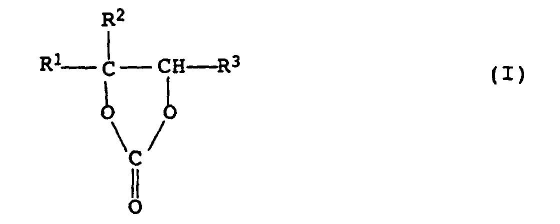 where R¹ is hydrogen or an organic radical having up to 40 carbon atoms and R² and R³ are each hydrogen or C₁-C₄-alkyl, in which case R² and R³ may also combine to form a five- or six-membered ring,
by reaction of an oxirane of the general formula II 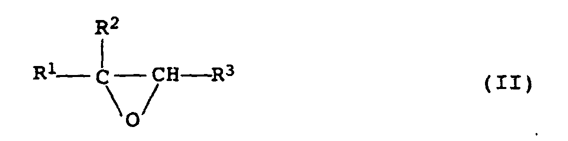 with carbon dioxide in the liquid phase in the presence of a catalyst, which comprises conducting the reaction in a two-part reactor in whose first part the reaction is taken with backmixing to a conversion of not less than 80%, especially not less than 90%, of the oxirane II and in whose second part the reaction is completed under nonbackmixing conditions, and passing the carbon dioxide in countercurrent to the oxirane II in the entire reactor.

2. A process as claimed in claim 1, wherein the fresh oxirane II and the fresh catalyst are fed into the first part of the reactor and the fresh carbon dioxide is fed into the second part of the reactor.

3. A process according to claim 1 or 2, wherein the reaction in the first part of the reactor is carried out isothermally by removing the heat of reaction.

4. A process as claimed in claim 3, wherein the heat of reaction is removed by an internal or external heat exchanger having a volume flow rate of from 30 to 500 times the throughput through the reactor.

5. A process as claimed in any of claims 1 to 4, wherein the reaction is completed up to a conversion of not less than 99%, especially not less than 99.5%, of the oxirane II in the second part of the reactor.

6. A process as claimed in any of claims 1 to 5, wherein the reactor is operated at from 2 to 50 bar, especially at from 5 to 40 bar, in both parts.

7. A process as claimed in any of claims 1 to 6, wherein the reactor is operated at from 70 to 150°C, especially at from 90 to 145°C, in both parts.

8. A process as claimed in any of claims 1 to 7, wherein the oxirane II and the carbon dioxide are used in a molar ratio of from 1:1 to 1:1.05.

9. A process as claimed in any of claims 1 to 8, wherein the catalysts used are onium salts or metal salts or mixtures thereof.

10. A process as claimed in claim 9, wherein the catalysts used are alkali metal iodides, alkali metal bromides, tetraalkylammonium iodides, tetraalkylammonium bromides, halides of divalent metals or mixtures thereof.

11. A process as claimed in any of claims 1 to 10 for producing 1,3-dioxolan-2-ones I where R¹ is hydrogen or C₁-C₁₈-alkyl.

12. A process as claimed in claim 11 for producing ethylene carbonate or propylene carbonate.

## Revendications

1. Procédé de préparation en continu de 1,3-dioxolane-2-ones de formule générale I 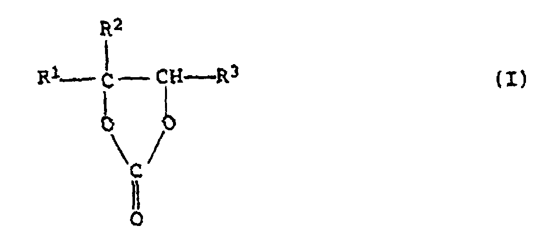 dans laquelle R¹ représente un atome d'hydrogène ou un groupe organique possédant jusqu'à 40 atomes de C, et R² et R³ représentent chacun un atome d'hydrogène ou un groupe alkyle en C₁ à C₄, et R² et R³ étant éventuellement liés l'un à l'autre en formant un cycle à cinq ou six éléments, au moyen d'une réaction d'un oxirane de formule générale II 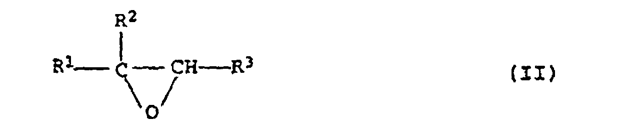 avec du dioxyde de carbone, en phase liquide, en présence d'un catalyseur, **caractérisé en ce que** l'on réalise la réaction dans un réacteur à deux parties, en réalisant la réaction, dans sa première partie, en remélangeant, jusqu'à la conversion de l'oxirane II à au moins 80%, en particulier à au moins 90%, et en achevant la réaction, dans des conditions sans remélange, dans la deuxième partie, où l'on amène le dioxyde de carbone, dans la totalité du réacteur, à contre-courant par rapport à l'oxirane II.

2. Procédé selon la revendication 1, **caractérisé en ce que** l'on introduit, dans la première partie du réacteur, l'oxirane frais II ainsi que le catalyseur frais, et, dans la deuxième partie du réacteur, le dioxyde de carbone frais.

3. Procédé selon la revendication 1 ou 2, **caractérisé en ce que** l'on réalise la réaction, dans la première partie du réacteur, de manière isothermique en dissipant la chaleur de réaction.

4. Procédé selon la revendication 3, **caractérisé en ce que** l'on dissipe la chaleur de réaction au moyen d'un échangeur de chaleur interne ou externe présentant un débit volumique de 30 fois à 500 fois supérieur au débit à travers le réacteur.

5. Procédé selon les revendications 1 à 4, **caractérisé en ce que** l'on achève la réaction, dans la deuxième partie du réacteur, jusqu'à ce la conversion de l'oxirane II à au moins 99%, en particulier à au moins 99,5%.

6. Procédé selon les revendications 1 à 5, **caractérisé en ce que** l'on fait fonctionner les deux parties du réacteur sous une pression allant de 2 bars à 50 bars, en particulier de 5 bars à 40 bars.

7. Procédé selon les revendications 1 à 6, **caractérisé en ce que** l'on fait fonctionner les deux parties du réacteur à une température allant de 70°C à 150°C, en particulier de 90°C à 145°C.

8. Procédé selon les revendications 1 à 7, **caractérisé en ce que** l'on met en oeuvre l'oxirane II et le dioxyde de carbone dans un rapport molaire allant de 1:1 à 1:1,05.

9. Procédé selon les revendications 1 à 8, **caractérisé en ce que** l'on met en oeuvre, en tant que catalyseur, des sels d'onium ou des sels métalliques ou bien des mélanges de ceux-ci.

10. Procédé selon la revendication 9, **caractérisé en ce que** l'on met en oeuvre, en tant que catalyseur, les iodures de métal alcalin, les bromures de métal alcalin, les iodures de tétraalkylammonium, les bromures de tétraalkylammonium, les halogénures de métaux bivalents ou bien des mélanges de ceux-ci.

11. Procédé selon les revendications 1 à 10 pour la préparation des 1,3-dioxolane-2-ones I, dans lesquelles le groupe R¹ représente un atome d'hydrogène ou un groupe alkyle en C₁ à C₁₈.

12. Procédé selon la revendication 11 pour la préparation du carbonate d'éthylène ou du carbonate de propylène.
